# EUROPÄISCHE PATENTSCHRIFT

(11) **EP 0 299 238 B1**
(45) Veröffentlichungstag und Bekanntmachung des Hinweises auf die Patenterteilung: **07.01.1993**
(21) Anmeldenummer: 88109888.3
(22) Anmeldetag: 22.06.1988
(51) Int. Cl.: C23C 24/10, B22F 7/00, A61F 2/30, A61L 27/00, C23C 24/00

(54) **Verfahren zur Beschichtung von Prothesen aus Titan und Titanlegierungen**
Process for coating titanium or titanium alloy prostheses
Procédé d'application d'un revêtement sur des prothèses en titane ou alliage de titane

(30) Priorität: 17.07.1987 DE 3723650
(43) Veröffentlichungstag der Anmeldung: 18.01.1989
(73) Patentinhaber: Fried. Krupp Gesellschaft mit beschränkter Haftung, 45143 Essen (DE)
(72) Erfinder: Mohs, Rudolf, Dr.rer.nat., D-4300 Essen 16 (DE); Bensmann, Günter, Dr.-Ing., D-4300 Essen 1 (DE)
(74) Vertreter: Vomberg, Friedhelm, Dipl.-Phys.

(56) Entgegenhaltungen:
- FR-A- 2 342 402
- FR-A- 2 398 118
- FR-A- 2 520 265
- GB-A- 1 128 228
- US-A- 4 612 160

## Beschreibung

Derzeit werden Endoprothesen überwiegend mit Knochenzement in dem vorbereiteten Prothesenlager fixiert. Diese Befestigungsmethode stößt in letzter Zeit zunehmend auf Kritik, da das System metallische Prothese/Knochenzement/Knochen keine ausreichende Dauerfestigkeit besitzt, was dazu führt, daß die so implantierten Prothesen lockern.

So ist aus der US-A-4 612 160 ein Verfahren zur Herstellung einer Prothese bekannt, bei dem ein Substratkörper an der Oberfläche gereinigt und in einer Form ausgerichtet wird, in die in dem Zwischenraum zwischen dem Forminnenmantel und dem Substratkörper Metallpulver eingefüllt und verdichtet wird. Nach einem Vorsintern wird der ganz oder teilweise beschichtete Metallsubstratkörper einem abschließenden Sintern unterzogen.

Aus diesem Grund versucht man seit einiger Zeit, Prothesen zementlos im Knochen zu fixieren. Um in diesem Fall einen guten Verbund zwischen Knochen und Implantat zu erreichen, ist man bestrebt, Prothesen mit einer strukturierten Oberfläche herzustellen. Solche Prothesen können grundsätzlich durch Feinguß oder durch anschließendes körniges Beschichten mit arteigenem Material hergestellt werden. Im Falle der Prothesen aus Titanlegierungen wird, soweit bekannt, grundsätzlich so verfahren, daß auf die geschmiedeten oder durch spanabhebende Bearbeitungsverfahren aus z.B. gewalztem Halbzeug herausgearbeiteten Prothesen in einem nachgeschalteten Fertigungsschritt die Struktur aufgesintert wird.

Der Nachteil bei diesem Verfahren ist, daß eine ausreichende Befestigung von z.B. Titankugeln auf einen Titanschaft durch sinternde Wärmebehandlung erst bei Temperaturen erzielt werden kann, die deutlich oberhalb der α-β-Transustemperatur liegt. Ein Beschichten oberhalb dieser Temperatur führt dazu, daß der Werkstoff grobkörnig wird und seine im Ausgangszustand sehr guten mechanischen Eigenschaften verliert.

So sind z. B. in St. A. Cook, Fatigue properties of carbon- and porous-coated Ti-6Al-4V alloy, Journal of Biomedical Materials Research, Vol. 18, 2. 497-512, 1984, Untersuchungen der Dauerbiegewechselfestigkeit an unbehandelten und beschichteten Proben aus der häufig als Implantatwerkstoff verwendeten Titanlegierung Ti-6Al-4V beschrieben, die beweisen, daß die Dauerbiegewechselfestigkeit der unbehandelten Titanlegierung von 617 N/mm² durch die Beschichtung durch sinternde Wärmebehandlung auf 138 N/mm² absinkt. Das Absinken der Dauerfestigkeit ist dabei auf die Überlagerung von zwei Effekten zurückzuführen: Einerseits auf die Kornvergröberung der Wärmebehandlung oberhalb der Umwandlungstemperatur und andererseits auf die durch die Oberflächenstruktur bedingte Kerbwirkung. Es wurden deshalb dort auch Proben untersucht, die zwar dem Temperaturzyklus der Wärmebehandlung des Beschichtungsvorgangs unterworfen, aber nicht beschichtet wurden. Bei diesen Untersuchungen wurde eine Dauerbiegewechselfestigkeit von 377 N/mm² festgestellt. Damit ist bewiesen, daß ein großer Anteil des Absinkens der Dauerbiegewechselfestigkeit von Titanlegierungen auf die Kornvergröberung bei dem oberhalb der α-β-Transustemperatur durchgeführten Beschichtungsprozeß zurückzuführen ist.

Aufgabe der vorliegenden Erfindung ist folglich ein Verfahren, bei dem der Beschichtungsprozeß unterhalb der α-β-Transustemperatur durchgeführt und trotzdem ein fester Verbund zwischen Struktur und Grundwerkstoff erreicht wird, so daß eine auf diese Weise beschichtete Prothese eine wesentlich höhere Dauerbiegewechselfestigkeit hat, als bisher.

Diese Aufgabe wird durch das im Anspruch 1 beschriebene Verfahren gelöst. Beispielsweise werden nach der Erfindung die auf die Prothesenoberfläche aufzubringenden kleinen Kugeln, mit einem bevorzugten definierten Durchmesser zwischen 500 bis 800 µm, vor dem Beschichtungsprozeß mit einer dünnen Schicht eines Werkstoffes überzogen, der mit dem Werkstoff der Prothesen und der Teilchen unterhalb der α-β-Transustemperatur eine flüssige Phase bildet.

Besonders vorteilhaft ist das Verkupfern der Teilchen.

Das Aufsintern der Teilchen geschieht vorzugsweise unter Vakuum oder einer Schutzgasatmosphäre, die wasserstoff-, sauerstoff- und stickstofffrei ist. Für verkupferte Teilchen ist eine Aufsinterungstemperatur von ca. 880°C bis 950°C besonders günstig. Der eigentliche Sinterungsprozeß dauert vorteilhafterweise 1 bis 10 Stunden. Besonders günstig ist die Fixierung der Teilchen vor dem Aufsintern mit einem geeigneten Kunststoffkleber. Das hat zur Folge, daß sich die Teilchen während des Beschichtungsvorganges nicht verschieben.

### Der Gegenstand der Erfindung wird am folgenden Beispiel erläutert:

Die z.B. durch Verdüsen hergestellten Titanlegierungskugeln von ca. 500 µm Durchmesser werden mit einer dünnen, ca. 2 µm dicken Schicht Kupfer überzogen. Danach werden sie auf dem Titanlegierungskörper zweilagig mit einem Kunststoffkleber mit Aceton verdünnt fixiert und unterhalb der α-β-Transustemperatur im Vakuum bei 920°C 2 Stunden geglüht. Während dieses Glühprozesses bildet sich eine flüssige TiCu-Phase, die an den ursprünglich punktförmigen Kontaktstellen zwischen den Ti-Kugeln und zwischen den Ti-Kugeln und dem Titan- oder Titanlegierungskörper durch Adhäsion verbreiterte Kontaktzonen bildet, die zu einer guten Haftung zwischen den Ti-Kugeln untereinander und den Ti-Kugeln und dem Titan- oder Titanlegierungskörper führt.

In Abhängigkeit von der Dauer dieses Sinterprozesses diffundiert das Cu unter Bildung diverser Phasen in die Kugeln und in den Titan- oder Titanlegierungskörper ein und kann danach an der Oberfläche nur noch in sehr geringen Konzentrationen nachgewiesen werden.
Soll der Titan- oder Titanlegierungskörper nur einlagig beschichtet werden, genügt es, nur diesen Körper zu verkupfern.

## Patentansprüche

1. Verfahren zum Aufsintern von arteigenen Teilchen auf die Oberfläche von Prothesenschäften aus Titan oder Titanlegierungen,
**dadurch gekennzeichnet,**
daß die Teilchen vor dem Aufsintern auf die Oberfläche des Prothesenschaftes mit einem Werkstoff beschichtet werden, der unterhalb der α-β-Transustemperatur des Titans oder der Titanlegierung des Prothesenschaftes und der Teilchen eine flüssige Phase bildet und daß der Prothesenschaft nach Fixierung der Teilchen auf seiner Oberfläche gesintert wird.

2. Verfahren nach Anspruch 1, dadurch gekennzeichnet, daß die Teilchen vor dem Aufsintern mit Kupfer beschichtet werden.

3. Verfahren nach einem der Ansprüche 1 oder 2, dadurch gekennzeichnet, daß das Aufsintern der Teilchen unter Vakuum durchgeführt wird.

4. Verfahren nach einem der Ansprüche 1 oder 2, dadurch gekennzeichnet, daß das Aufsintern der Teilchen in einer wasser-, sauerstoff- und stickstofffreien Schutzgasatmosphäre durchgeführt wird.

5. Verfahren nach einem der Ansprüche 1 oder 2, dadurch gekennzeichnet, daß die Teilchen vor dem Aufsintern mit einem Kunststoffkleber auf dem Prothesenschaft fixiert werden.

## Claims

1. A process for sintering particles of titanium or titanium alloys on to the surface of prosthesis shafts of titanium or titanium alloys, characterized in that prior to being sintered on to the surface of the prosthesis shaft the particles are coated with a material which forms a liquid phase below the α-β transition temperature of the titanium or the titanium alloy of the prosthesis shaft, the prosthesis shaft being sintered after the fixing of the particles on its surface.

2. A process according to claim 1, characterized in that prior to being sintered on the particles are coated with copper.

3. A process according to claims 1 or 2, characterized in that the particles are sintered on in vacuo.

4. A process according to one of claims 1 or 2, characterized in that the particles are sintered on in a protective gas atmosphere free from hydrogen, oxygen and nitrogen.

5. A process according to one of claims 1 or 2, characterized in that prior to being sintered on the particles are fixed to the prosthesis shaft with a plastics glue.

## Revendications

1. Procédé d'agglomération par frittage de particules caractéristiques de l'espèce, sur la surface de tiges de prothèses en titane ou alliage de titane, caractérisé en ce que les particules sont couvertes, avant leur agglomération par frittage sur la surface de la tige de la prothèse, d'une matière qui forme une phase liquide au-dessous de la température transus α-β du titane ou de l'alliage de titane de la tige de la prothèse et des particules, et en ce que la tige de la prothèse est frittée après fixation des particules sur sa surface.

2. Procédé selon la revendication 1, caractérisé en ce que les particules sont couvertes de cuivre avant l'agglomération par frittage.

3. Procédé selon l'une des revendications 1 ou 2, caractérisé en ce que l'agglomération des particules par frittage s'effectue sous vide.

4. Procédé selon l'une des revendications 1 ou 2, caractérisé en ce que l'agglomération des particules par frittage s'effectue dans une atmosphère de gaz protecteur anhydre, exempt d'oxygène et non azoté.

5. Procédé selon l'une des revendications 1 ou 2, caractérisé en ce que les particules sont fixées sur la tige de la prothèse avec une colle synthétique, avant l'agglomération par frittage.
